# EUROPEAN PATENT APPLICATION

(11) **EP 2 033 526 A1**
(43) Date of publication of application: **11.03.2009**
(21) Application number: 07017570.8
(22) Date of filing: 07.09.2007
(51) Int. Cl.: A23C 9/152, A23K 1/18, A23L 1/30, A23L 1/318, A23L 1/325, A23L 2/52, A61K 31/352, A61P 9/12, A61P 3/06

(54) **Nutritional products comprising pomegranate extracts containing ellagitannins and their use**

(71) Applicant: Probelte Pharma, S.A., 30100 Espinardo Murcia (ES)
(72) Inventor: López Más, José A, 30840 Alhama de Murcia Murcia (ES); Streitenberger, Sergio A., 30120 El Palmar Murcia (ES); Peñalver Mellado, Marcos, 30007 Murcia Murcia (ES); Pedreño López, Yolanda, 30004 Murcia Murcia (ES); Martinez Ortiz, Pedro, 30150 La Alberca Murcia (ES)
(74) Representative: Gislon, Gabriele

(57) **Abstract**

The present invention relates to new pomegranate extracts compositions completely free of any kind of organic solvent, soluble in cold water in high concentration, with very low sugar content and specially standardized to punicalagins to mimics the natural profile of phytochemicals present in the natural pomegranate juice obtained from whole pomegranate fruits, and its addition to nutritional products producing functional foods and beverages with increased antioxidant capacity to be used as a source of punicalagins for preventing or treating hypercholesterolemia and/or hypertension, thanks to the nutritional supply of a punicalagins rich composition.

## Description

The present invention relates to nutritional products comprising pomegranate extracts containing ellagitannins and their use. Namely, the invention relates to use of both pomegranate extracts and the nutritional products functionalized with pomegranate extracts for maintaining normal levels or reducing until normal levels both the concentration of cholesterol constituents in the blood and the arterial blood pressure. According to that, pomegranate extract derived from whole pomegranate fruit is added to beverages and human or veterinary food products, which results in an increase of the level of anti-oxidants, particularly of ellagitannins and more particularly of punicalagins. More particularly, the invention relates to the functionalization or fortification of nutritional products to be used as a source of punicalagins for preventing or treating hypercholesterolemia and/or hypertension, thanks to the nutritional supply of a punicalagins rich composition.

According to the supplement published by the British Heart Foundation: European cardiovascular disease statistics, 2005 edition, CVD are the main cause of death in Europe: accounting for over 4.35 million deaths each year. Coronary heart disease (CHD) by itself is the single most common cause of death in Europe: accounting for 1.95 million deaths in Europe each year.

Total costs of CVD amounts to 169 billion euros, of which 105 billion euros are for treating CVD in the European Union and 64 billion euros are due to lost productivity and the cost of informal care.

Atherosclerosis is the main cause of CVD and the most frequent cause of CHD and has been demonstrated that arterial hypertension, high levels of triglycerides and total cholesterol in the blood, and smoking are factors that contribute to the development of this affection.

Numerous studies have demonstrated that in vivo oxidation of LDL plays a central role in the development of atherosclerosis (Knight, 1995; Witzum, 1994).

In both, laboratory and clinical studies, pomegranate shows promising results in preventing cardiovascular diseases. Scientists provide evidences about pytochemicals from pomegranate work through several mechanisms: by inhibiting the oxidation of LDL and supporting the synthesis and activity of nitric oxide (De Nigris et al, Nitric Oxide. 2006), reducing oxidative stress and inflammatory damage in blood vessels (De Nigris et al. Cardiovasc. Res. 2007; Rozenberg et al.

Atherosclerosis. 2006), decreasing activity of angiotensin converting enzyme (Aviram et al, Atherosclerosis. 2001). Mounting evidence suggest that compounds in pomegranate known as punicalagins are cardioprotective by virtue of their powerful antioxidant and anti-inflammatory affects.

Studies have shown that pomegranate juice has the higher antioxidant properties than any other drink, such as blueberry juice, cranberry juice, green tea or red wine. Punicalagins α and β anomers are predominant ellagitannins in pomegranate juice, and the major responsible of antioxidant properties of the juice. Nevertheless, research pointed out that combination of punicalagins α and β with other ellagitannins such as ellagic acid and its glycosidated derivatives and other minor pomegranate ellagitannins produces and important synergistic effect enhancing healthy benefits of the juice. Punicalagins α and β anomers are mainly present in pomegranate husk, and they pass to the primary juice during pressing for juice extraction. Punicalagins α and β have the following formula:

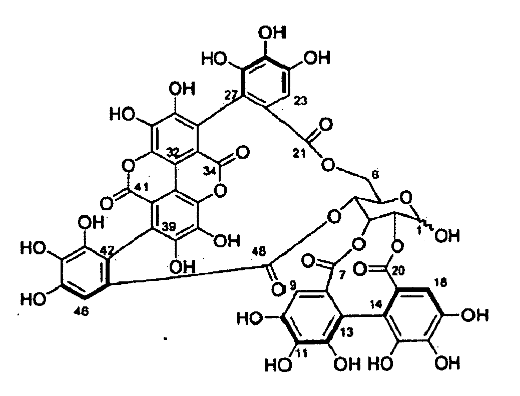

WO02/056899 relates to compositions and methods for reducing oxysterols in the blood and normalizing cholesterol and blood pressure. This patent discloses compositions that preferably comprise: Noni (Morinda Citrifolia) extract, red wine extract, prune extract, blueberry extract, pomegranate extract, apple extract, and an enzyme mixture. Patent claims a method to maintain or reduce oxysterols in blood comprising the step of administering the above mentioned composition wherein said pomegranate extract comprises about 40 % ellagic acid and about 60 % polyphenols.

Moreover, when free ellagic acid is administrated orally, it is poorly absorbed.

US 2006/0211635 discloses purification of pomegranate ellagitannins from fruit husk, a by-product of the commercial juice industry. This process has the drawbacks of requiring the use, for elution of ellagitannins adsorbed onto a resin surface, of organic solvents, possibly toxic as methanol, and of generating an extract than doesn't contain several phytochemicals (anthocyanins, etc) exclusively present in the pomegranate juice fraction, when in fact the juice is the product consumed by the people, to which recent studies attribute the healthy benefits obtainable from pomegranate.

WO2006/127832 discloses a method to produce a pomegranate extract from by-products of the juice industry. The process described, includes several thermical treatments where the extract is subjected to heating (2 h at 43-71 °C and pasteurization). Pomegranate juice pasteurization produces free ellagic acid trough degradation of larger ellagitannins, such as punicalagins, diminishing the health benefits of drinking the juice. These steps generate several by-products that are difficult to remove and, in addition, the use of pomegranate solids coming from the juice industry generates an extract than doesn't contain some phytochemicals (minor ellagitannins, anthocyanins, etc) exclusively present in the pomegranate juice fraction. For this reason WO2006/127832 discloses the combination of pomegranate extract and juice, that in fact provides a broad spectrum of the phytochemicals present in the whole pomegranate, but has the disadvantage of the high caloric value due to the sugars present in both the juice and the extract. Additionally, a high sugar content makes difficult the industrial processing of such extracts because the purification of the punicalagins is difficult and the solid extracts stick to the walls of the drying equipments. The patent claims related to beverages and food products comprising pomegranate extract are referred to extracts prepared from pomegranate solids selected from the group consisting of the pericarp, inner membrane and seeds but didn't refer to extracts prepared from whole pomegranate fruits.

US Application 20070178180 relates to pomegranate extracts and methods of using thereof, and specifically to methods of using pomegranate extracts for causing regression in lesions due to arteriosclerosis in humans. This document presents a human study with 19 patients with carotid artery stenosis (CAS). Ten of these patients consumed one glass of pomegranate juice every day during 12 months. Authors of the document conclude that results of the human study demonstrate statistically significant anti-atherogenic effects of pomegranate juice consumption in patients with CAS. Both the pomegranate juice used and the pomegranate extracts prepared according the above mentioned application are prepared without any specific purification step.

US Application 20020012710 relates to pomegranate products useful in improving health. Patent claims a method for use the pomegranate seed oil, comprising administering the pomegranate seed oil to a subject to exert an effect on a physiologic process. Into such effect it is included an antioxidant effect. Additionally, this patent claims a method of retarding the process of atherosclerosis, comprising administering fermented pomegranate juice to a patient.

Summarizing, the above mentioned techniques provide pomegranate products that in spite of providing benefits to the health, are still too crude for certain technological applications of the functional food and beverage industry, which supposes some disadvantages such as:
- Regular consumption of the juice or the crude extracts supposes the uptake of a high caloric value due to the sugars present in both.
- Use of organic solvents to obtain some of the above mentioned pomegranate extracts.
- Standardization of some of the above mentioned pomegranate extracts is very far from the natural profile of phytochemicals (e.g. ellagitannins) existing in the natural pomegranate juice obtained from processing of the whole pomegranate fruit. It is to say for instance, some extracts have been standardized to 40 % ellagic acid instead to punicalagins.
- Such products are not completely water soluble, specially the extracts enriched in ellagic acid, meaning that its incorporation in beverages will produce turbidity making the product lees attractive for the consumers.
- free ellagic acid is poorly absorbed when administered orally.

In addition, punicalagins content is not standardized in most of the pomegranate products above mentioned.

It is an aim of the present invention to solve the above mentioned problems and to provide both pomegranate extracts containing ellagitannins, and nutritional products containing this added pomegranate extracts to be used for maintaining normal levels or reducing until normal levels both the concentration of cholesterol constituents in the blood and the arterial blood pressure, but avoiding non-pleasant alterations of properties of the fortified foods such us turbidity of the fortified beverages and increase of the caloric content. Additionally, the absence of any organic solvent in the extracts of the invention is advantageous both from the economical, and health points of view when compared with other pomegranate extracts containing residual solvents.

Such aim is achieved by means of the present invention that provides functional foods and beverages with pomegranate extracts standardized to its punicalagins content.

In co-pending patent application EP07004765.9, that is hereto incorporated by reference, the present applicant described and claimed pomegranate extracts and a process for preparing the same, which aim at the production of ellagitannins- containing products to be used as a source of ellagitannins from the pomegranate in food, medical and cosmetic industries.

The process disclosed and claimed in EP 070047565.9 for producing a pomegranate extract from a suspension of blended fruit in water, comprises the steps of carrying out an extraction of the entire blended fruit in water at a temperature within the range of 4°C to 30°C, preferably of 8°C to 25°C, and at a pH within the range of 3.5 to 5.0. The extraction is carried out in a mill and the duration of said extraction step is within the range of 15 to 150 minutes.

The extraction mixture is then treated to remove solids and to obtain a clarified aqueous solution that is loaded in a chromatographic column of an adsorbent nonionic resin; the products retained in the chromatographic column are eluted by shifting the pH with a basic solution, and displacing the products with demineralised water. Preferably, said shifting of the pH is carried out with a sodium bicarbonate elution buffer solution.

The liquid product eluted from the resin is preferably concentrated by nanofiltration and water is removed until the product is in a solid form. By means of this method it is possible to obtain a product with a dramatically reduced content of sugars or with no sugars at all. Such a product was not known in the art, as per the above discussion of prior art.

The process of production of such solvent-free natural pomegranate extracts, discussed in co-pending patent application EP07004765.9, is simple, effective, and not expensive, providing both water soluble punicalagins, rather than solvent soluble ellagic acid, and a broad spectrum of different natural organic chemicals which are present in the whole pomegranate fruit and useful to improve activity of the main compounds of the extract.

An aspect of the invention relates to a process for preparing the functional foods and beverages with pomegranate extract, which comprises incorporating and mixing the pomegranate extract with the nutritional product to be functionalized.

A second aspect of the invention relates to nutritional products, functional foods and beverages, comprising the mentioned added pomegranate extract conferring they improved antioxidant capacity.

Finally, a third aspect of the invention relates to the use of the nutritional products, functional foods and beverages, in the prevention or treatment of hypercholesterolemia and/or hypertension.

As mentioned above, the first aspect of the invention relates to preparing the functional foods and beverages with pomegranate extract.

The compositions of such natural pomegranate extracts were discussed in corending patent application EP07004765.9 and are summarized in the following paragraph for better understanding of the present invention.

Compositions as obtained of the above mentioned co-pending invention are the ellagitannins containing products in solid form and are characterized by having the following characteristics:
- a punicalagins content (as % w/w) of not less than 5% and preferably of not less than 30% and more preferably of not less than 50%.
- a purity of not less than 55% and preferably of not less than 70% and more preferably of not less than 80%.
- a ellagic acid (free acid) content (as % w/w) of not more than 3% and preferably of not more than 2% and more preferably of not more than 1.5%.
- a total phenol content (as % w/w gallic acid equivalent) of not less than 20% and preferably of not less than 30% and more preferably of not less than 35%.
- a solubility in water (as % w/v) of not less than 3% and preferably of not less than 7% and more preferably of not less than 10%.
- a residual organic solvent content of 0 ppm (i.e. the extract is free from any kind of organic solvent).
- a total sugar content (expressed in ppm) of not more than 500 and preferably of not more than 300 and more preferably of not more than 50.

The invention process of preparing a functional (or fortified) food or beverage with pomegranate extract, consists in incorporating and mixing the pomegranate extract of the above type with the food product to obtain a so-called functional or functionalized food (or beverage) product, or functionalized nutritional product. The addition of the pomegranate extract to the nutritional product is obtained by mixture and homogenisation according to the technological process of manufacturing each nutritional product which will be functionalized.

Preferably natural extracts according to the co-pending patent application EP07004765.9 are used, so as to avoid any trace of organic solvents in the final product as well as to avoid the high caloric value due to the sugars present in other pomegranate products, especially in view of their use in the prevention and treatment of hypercholesterolemia and arterial hypertension.

Functionalization (fortification) of solid nutritional products for human or animal consumption comprises the step of adding pomegranate extract, usually as early as possible, in the production process to facilitate its incorporation and homogeneous distribution whether the ingredients are pre-mixed with other dry ingredients or dispersed in water, according to the known art.

Functionalization (fortification) of beverages comprises the step of incorporating and dissolving the pomegranate extract during the technical steps of beverage preparation. In a general way, pomegranate extract powder and optionally further hydrophilic components are added, in the weight ratio desired, to the beverage and completely dissolved, under very controlled parameters of temperature and agitation. This step is preferably carried out straight afterwards the beverage is pasteurized or food acids and/or food preservatives are added, to avoid spoilage.

A second aspect of the invention relates to a nutritional product, to which the pomegranate extract defined above has been incorporated producing functional foods and beverages. In the context of the present invention, by the expression "nutritional product" it is understood any food product (including beverages) for human or animal consumption.

The nutritional products of the invention preferably comprise a concentration of the pomegranate extract of the invention equivalent to a punicalagins content ranging from 0.005 % to 0.25 % (w/w). A preferred range comprises an amount of extract equivalent to a punicalagins content ranging from 0.01 % to 0.05 % (w/w). Nutritional products containing this amount of added pomegranate extract can be: fruit-flavoured beverages, orange flavoured beverages, lemon-lime flavoured beverages, root beer, cola, fruit juices, fruit-flavoured, or fruit-containing beverages, vegetable juices or vegetable containing beverages, sport drinks, energy drinks, flavoured water, beverages comprising a dairy component, milk, milk shake, fermented milk, flavoured milk horchata, beer, beer-type sparkling drink, wine, wine-type drink, coffee, coffee-based beverages, tea, tea-based beverages, infusions, to name a few, technologically modified derivatives of the above mentioned beverages or mixtures of two or more of the same.

Another preferred range comprises an amount of extract equivalent to a punicalagins content ranging from 0.02 % to 0.20 % (w/w). Nutritional products containing this amount of added pomegranate extract can be:
- dairy product such us, yogurts, ice creams, cheeses.
- vegetable, marine or fish oils obtained from various sources such us olive (extra virgin olive oil, virgin olive oil, lampante olive oil, refined olive oil, crude olive-pomace oil, refined olive-pomace oil), sunflower, corn, soya, flax seed, almond, canola, safflower, palm, coconut, rapeseed, algae, krill, menhaden, anchovy, tuna.
- meat or poultry product such us, chicken, turkey, duck, pork, beef, prepared as sausages, smoke-dried or cold meat, such as cured ham, boiled ham, smoked ham, mortadella, salami, pâté, canned precooked meats.
- a bakery product or a pasta-based product such us breads, bagels, biscuits, cookies, cakes, pastries, pies, macaroni, spaghetti.
- a vegetable or fruit conserve such as canned tomatoes, canned artichokes, canned pineapple, canned peach, jams, marmalades, jellies, pickles.
- a canned seafood or canned fish such as shrimp, lobster, squid, crab, mussel, cockle, tuna, sardine.
- a snack or sweet such as almonds, peanuts, pistachios, walnuts, popcorns, chocolates, chewing gums, candies.
to name a few, technologically modified derivatives of the above mentioned food products or mixtures of two or more of the same.

Another preferred range comprises an amount of extract equivalent to a punicalagins content ranging from 0.025 % to 0.25 % (w/w). Nutritional products containing this amount of added pomegranate extract can be: pet food such us canned wet foods, dry pet foods, semi-moist foods, snacks, to name a few, technologically modified derivatives of the above mentioned pet foods or mixtures of two or more of the same.

Finally a third aspect of the invention relates to the use of the nutritional products of the invention, functionalized with pomegranate extract, in the prevention or treatment of hypercholesterolemia and arterial hypertension. The uptake of functional foods and beverages with pomegranate extract is preferable in accordance with a predetermined regimen, which preferably would be 2-3 servings daily, and may be over an extended period of time as a chronic treatment, and could last from one year or more, including the life of the consumer.

In the context of the present invention, by the expression "serving" is to be understood as:
- a helping, i.e. an individual quantity of food or drink taken as part of a meal, or
- for one food, the amount a person, or an animal, would be likely to eat during a meal.

According to the invention, this portion of a functional nutritional product containing the amount of the pomegranate extract of the present invention in sufficient quantity to achieve the intended purpose, when consumer take 2-3 servings per day in accordance with a predetermined regimen. In one embodiment, punicalagins are present in the functional nutritional product composition in an amount within the range of 5 mg to 5000 mg per serving, preferably 15 to 500 mg and more preferably of 40 mg to 200 mg per serving.

According to another preferred embodiment of the present invention, the fortified, i.e. functionalised, nutritional product contains the above mentioned extracts and hydroxytyrosol. Preferably, the hydroxytyrosol is obtained by extraction according to co-pending EP applications n. 07001791.8 and 07014390.4.

The invention will now be further disclosed in greater detail with reference to the enclosed following non-limiting examples and drawings wherein:

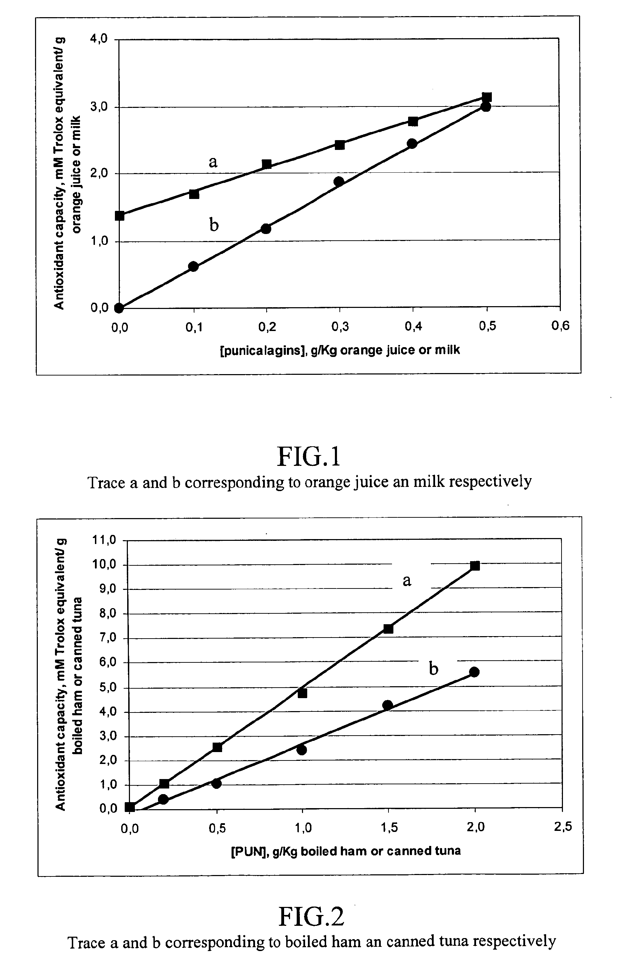

- figures 1-3 are graphs showing the linear correlation between the antioxidant capacity of different nutritional products obtained according the present invention (see examples 1 to 5) and its content in punicalagins measured by HPLC as a result of its fortification with increasing quantities of pomegranate extracts obtained according the present invention.

### EXAMPLE 1

### Preparation of a functional juice with pomegranate extract

### Processing technology:

The final product was prepared from a concentrated juice by addition of water and water soluble ingredients. Then, pomegranate extract was added and mixed and the resulting product was pasteurised and homogenized. Finally, the product was cooled and packaged.

The content of pomegranate extract ranges from 200 to 1000. mg/Kg. (such pomegranate extract having a punicalagins content of 50 % w/w). Measurement of antioxidant capacity was carried out as follows.

Six samples of functional juice were prepared adding to 40 g of orange juice 0, 8, 16, 24, 32 y 40 mg of pomegranate extract (such pomegranate extract having a punicalagins content of 50 % w/w) and aliquots of every one of them were used to measure antioxidant capacity (radical ABTS absorption capacity measured at 734 nm using Trolox as standard) and punicalagins content by HPLC. When results were plotted (see figure 1, plot a) as antioxidant capacity vs. concentration of punicalagins a linear correlation was obtained. Concretely antioxidant capacity of the non-functionalized juice was 1.39 mM Trolox equivalent per gram and antioxidant capacity of the functional juice with the highest weight ratio used in this example was 3.13 mM Trolox equivalent per gram, meaning that a 2.3 fold increase in the antioxidant capacity of the juice was obtained.

### EXAMPLE 2

### Preparation of a functional milk based product with pomegranate extract

### Processing technology:

All solid ingredients were mixed with the liquid milk and water. Then, pomegranate extract was added and homogenised in the absence of oxygen. The resulting dairy product was then subjected to U. H. T. treatment (150 C for 4 to 6 seconds) and finally packaged in the absence of oxygen.

The content of pomegranate extract ranges from 200 to 1000. mg/Kg. (such pomegranate extract having a punicalagins content of 50 % w/w). Measurement of antioxidant capacity was carried out as follows.

Six samples of functional milk were prepared adding to 40 g of milk 0, 8, 16, 24, 32 y 40 mg of pomegranate extract (such pomegranate extract having a punicalagins content of 50 % w/w) and aliquots of every one of them were used to measure antioxidant capacity (radical ABTS absorption capacity measured at 734 nm using Trolox as standard) and punicalagins content by HPLC. When results were plotted (see figure 1, plot b) as antioxidant capacity vs. concentration of punicalagins a linear correlation was obtained. Concretely antioxidant capacity of the non-functionalized milk based product was 0 mM Trolox equivalent per gram and antioxidant capacity of the functional milk based product with the highest weight ratio used in this example was 2.97 mM Trolox equivalent per gram.

### EXAMPLE 3

### Preparation of a functional boiled ham with pomegranate extract

### Processing technology:

Pomegranate extract is previously blended with the mixture of industrial ingredients (salt, glucose syrup, sugar, several food additives) and this mixture incorporated into the brine solution. Straight afterwards the brine solution is forced through the holes into the ham piece. Several massages, under vacuum and temperature below 10 °C, are made to the ham piece. Then, ham piece is macerated at 4-6 °C during at least 48 h. After that piece is again massaged and packed under vacuum into a cooking bags that are put into a mold. Now the ham piece is baked in a steam oven until the temperature in the centre of the piece reach 65 °C. Straight afterwards the piece of boiled ham is cooled down and stored at 5 °C. Finally after 24 h the mold is removed.

The content of pomegranate extract ranges from 400 to 4000. mg/Kg. (such pomegranate extract having a punicalagins content of 50 % w/w).

Measurement of antioxidant capacity was carried out as follows.

Six samples of functional boiled ham were prepared adding to 3 g of boiled ham 0, 1.2, 3, 6, 9 y 12 mg of pomegranate extract (such pomegranate extract having a punicalagins content of 50 % w/w) and aliquots of every one of them were used to measure antioxidant capacity (radical ABTS absorption capacity measured at 734 nm using Trolox as standard) and punicalagins content by HPLC. When results were plotted (see figure 2, plot a) as antioxidant capacity vs. concentration of punicalagins a linear correlation was obtained. Concretely antioxidant capacity of the non-functionalized boiled ham was 0.11 mM Trolox equivalent per gram and antioxidant capacity of the boiled ham with the highest weight ratio used in this example was 9.93 mM Trolox equivalent per gram, meaning that a 90.3 fold increase in the antioxidant capacity of the boiled ham was obtained.

### EXAMPLE 4

### Preparation of a functional canned tuna with pomegranate extract

### Processing technology:

Pomegranate extract is added as early as possible in the production process to facilitate its incorporation and homogeneous distribution whether the ingredients are pre-mixed with other dry ingredients or dispersed in water, according to the known art.

The content of pomegranate extract ranges from 400 to 4000. mg/Kg. (such pomegranate extract having a punicalagins content of 50 % w/w). Measurement of antioxidant capacity was carried out as follows.

Six samples of functional tuna were prepared adding to 3 g of tuna 0, 1.2, 3, 6, 9 y 12 mg of pomegranate extract (such pomegranate extract having a punicalagins content of 50 % w/w ) and aliquots of every one of them were used to measure antioxidant capacity (radical ABTS absorption capacity measured at 734 nm using Trolox as standard) and punicalagins content by HPLC. When results were plotted (see figure 2, plot b) as antioxidant capacity vs. concentration of punicalagins a linear correlation was obtained. Concretely antioxidant capacity of the non-functionalized canned tuna was 0 mM Trolox equivalent per gram and antioxidant capacity of the canned tuna with the highest weight ratio used in this example was 5.54 mM Trolox equivalent per gram.

### EXAMPLE 5

### Preparation of a functional semi-moist dog food with pomegranate extract

### Processing technology:

Pomegranate extract is added as early as possible in the production process to facilitate its incorporation and homogeneous distribution whether the ingredients are pre-mixed with other dry ingredients or dispersed in water, according to the known art.

The content of pomegranate extract ranges from 400 to 5000. mg/Kg. (such pomegranate extract having a punicalagins content of 50 % w/w). Measurement of antioxidant capacity was carried out as follows.

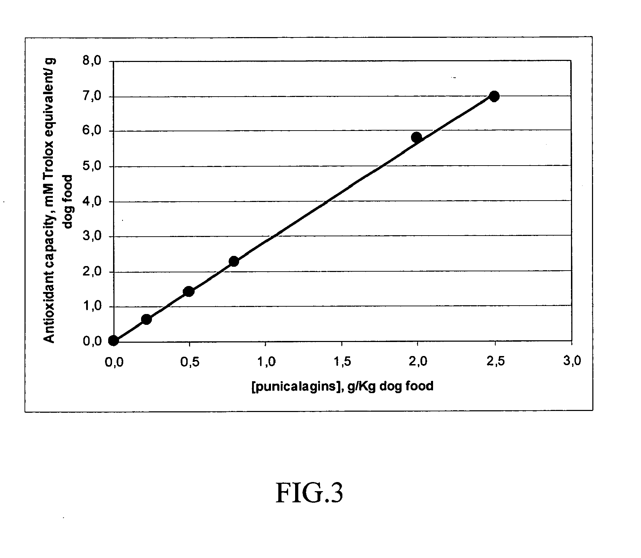

Six samples of functional dog food were prepared adding to 3 g of dog food 0, 1.3, 3, 4.8, 12 y 15 mg of pomegranate extract (such pomegranate extract having a punicalagins content of 50 % w/w) and aliquots of every one of them were used to measure antioxidant capacity (radical ABTS absorption capacity measured at 734 nm using Trolox as standard) and punicalagins content by HPLC. When results were plotted (see figure 3) as antioxidant capacity vs. concentration of punicalagins a linear correlation was obtained. Concretely antioxidant capacity of the non-functionalized dog food was 0.02 mM Trolox equivalent per gram and antioxidant capacity of the dog food with the highest weight ratio used in this example was 6.95 mM Trolox equivalent per gram, meaning that a 347.5 fold increase in the antioxidant capacity of the dog food was obtained.

### EXAMPLE 6

### Use of functionalized rat standard feed and water with pomegranate extract in the prevention or treatment of hypercholesterolemia.

### 6.1. Animals

Sixty-four male Sprage Dawley rats (weight aprox. 150-180 g) were obtained from Harlan Interfauna lberica SA (Barcelona, Spain) and maintained during all the experiment in the installations of the animalary service at the University of Murcia. The animals were randomly distributed into 8 experimental groups of 8 rats each one, and every 4 rats subgroup housed under standard conditions of lighting (day/night cycles of 12 h), temperature (18±1 °C) and humidity (60%).

### 6.2. Diets

The diets used in the study were as follows:
- NTROL DIET (C): solid standard rat diet (Panlab).
- ATHEROGENIC DIET (A): 95.5% standard rat diet (Panlab), 1.5% of cholesterol (Aldrich) and 3% of lard to induce atherosclerosis.
- ATHEROGENIC DIET + POMEGRANATE EXTRACT (P): 95.0% standard rat diet (Panlab), 1.5% of cholesterol (Aldrich), 3% of lard and 0.5 % pomegranate extract (punicalagins content: 50 % w/w).
- STANDARD + ORALLY GAVAGE (80 mg punicalagins/Kg) (G80): standard rat diet (Panlab). Additionally, functionalized water containing 80 milligram of punicalagins/kg of body weight were administered by oral gavages every day.
- STANDARD + ORALLY GAVAGE (160 mg punicalagins/Kg) (G160): standard rat diet (Panlab). Additionally, functionalized water containing 160 milligram of punicalagins/kg of body weight were administered by oral gavages every day.
- STANDARD + ORALLY GAVAGE (5 mg hydroxytyrosol/Kg) (G5): standard rat diet (Panlab). Additionally, fortified extra virgin olive oil containing 5 milligram of HT/kg of body weight (BW) were administered by oral gavages every day.
- STANDARD + COMBINED ORALLY GAVAGE (80 mg punicalagins + 5 mg hydroxytyrosol/Kg) (G80 + 5): standard rat diet (Panlab). Additionally, functionalized water containing 80 milligram of punicalagins/kg of body weight plus fortified extra virgin olive oil containing 5 milligram of hydroxytyrosol/kg of body weight were administered by oral gavages every day.

Drinking water and food were available ad libitum. Nevertheless, throughout the study and average food supply for animal was standardised to 15 g/day (real food intake per animal was unknown). Additionally, 80 mg of punicalagins/kg of body weight, 160 mg of punicalagins/kg of body weight or 80 mg of punicalagins + 5 mg hydroxytyrosol / kg of body weight were administered by oral gavages to animal groups G80, G160 and G80 + 5 respectively, every day. To avoid fat oxidation, atherogenic diet (A) and atherogenic diet + pomegranate extract (P) were kept at 4°C in the dark until use, and the non-consumed diet of past day was removed. The concentrations of punicalagins and/or hydroxytyrosol in the P, G80, G160 and G80 + 5 diets used in the study were measured by high performance liquid chromatography (HPLC).

### 7.3. Experimental design

The experimental design is shown in the following Table 1:

**Table 1.**

| GROUP | From day 1 to 30 | From day 31 to 60 | Remarks |
|---|---|---|---|
| 1 | Diet C | Diet C | |
| 2 | Diet A | Diet A | |
| 3 | Diet A | Diet C | |
| 4 | Diet P | Diet P | |
| 5 | Diet A | Standard Diet + G80 | 80 mg of punicalagins (as functionalized water) per kg of BW were administered daily by oral gavages. |
| 6 | Diet A | Standard Diet + G160 | 160 mg of punicalagins (as functionalized water) per kg of BW were administered daily by oral gavages. |
| 7 | Diet A | Standard Diet + G5 | 5 mg of hydroxytyrosol (as fortified evoo) per kg of BW were administered daily by oral gavages. |
| 8 | Diet A | Standard Diet + G80+5 | 80 mg of punicalagins (as functionalized water) + 5 mg of hydroxytyrosol (as fortified evoo) per kg of BW were administered daily by oral gavages. |

Animal groups CC, AA, AC, PP, AG80, AG160, AG5 and AG80 + 5 were fed for 2- months with diets C, A or P (groups CC, AA, or PP respectively), or for 1-month with the A-diet followed by a further month with diet C (group AC), or standard rat diet with orally gavage of 80 mg of punicalagins/kg of body weight, 160 mg of punicalagins/kg of body weight, 5 mg hydroxytrosol/kg of body weight or 80 mg of punicalagins + 5 mg hydroxytyrosol / kg of body weight (groups AG80, AG160, AG5 and AG80 + 5, respectively). At the end of the experiment, fasted animals were anesthetized and immediately after euthanasia intra-cardiac punction was made to collect blood in tubes. Serum was separated by centrifugation for analysis.

### 6.4. Determination of lipids, and total antioxidant capacity (TAC) in plasma.

The plasma concentrations of total cholesterol (TC), HDL-cholesterol (HDL-C) and triglycerides (TG) were determined by colorimetry using commercial kits purchased from Biosystems (Barcelona, Spain) according to the manufacturer's instructions.

The TAC of plasma was measured as described by Re et al (1999) using Trolox as standard. Fresh plasma was diluted in PBS and 20 microL were incubated in the dark with 980 microL of ABTS+ [2,2_-Azino-bis(3-ethylbenzthiazoline-6-sulfonic acid)] radical cation for 10 min at room temperature. After 10 minutes, absorbance was read at 734 nm. ABTS+ cation mother solution was prepared by addition of 440 microL of 14 mM potassium persulfate in water to 25mL of a 7mM solution of ABTS (Sigma A-1808) in water and incubation for 12-14 h in the dark. The working solution was obtained by dilution of the mother solution with PBS until the absorbance at 734 nm was 0.7±0.02.

### 6.5. Statistical analysis

The data are expressed as means±standard error of the means (S.E.M.), and were analyzed by one-way ANOVA. Differences between the groups were assessed by the Tukey test. Differences were considered significant when P-values were <0.05. The data was analysed using Sigma Stat software (Version 2.03).

### 6.6. Discussion.

High cholesterol/trilglycerides levels, low HDL cholesterol levels and specially oxidised LDLs are among risk factors associated with atherosclerosis and coronary heart disease. We have studied the effect that a functionalized nutritional product containing a pomegranate extract has on biomarkers for cardiovascular events, and compared it with similar nutritional products no-functiolized with pomegranate extract. Taking all the parameters measured into account, we conclude the regular administration of the nutritional product containing a pomegranate extract protects the cardiovascular system which, to a greater or lesser extent, prevents the occurrence of cardiovascular events and therefore may well be considered as a health promoting nutritional product.

### EXAMPLE 7

### Use of pomegranate extract in the treatment of hypertension.

### 7.1. Animals

Forty-two male Sprage Dawley rats (weight aprox. 150 at the init of the experiment) were obtained from Harlan Interfauna lberica SA (Barcelona, Spain) and maintained during all the experiment in the installations of the animalary service at the University of Murcia. The animals were randomly distributed into six experimental groups of 6 rats each one, housed under standard conditions of lighting (day/night cycles of 12 h), temperature (18±1 °C) and humidity (60%).

### 7. 2. Diet

During the acclimatation and experimental periods, rats were feed on a solid standard diet for rats (Panlab). Drinking water and food were available ad libitum. After an acclimatation period of 7 days treatments according the experimental design were started.

### 7.3. Experimental design

In order to determine effect of pomegranate extract an N-nitro-L-arginine-methylester (L-NAME) induced hypertension model was used After the acclimatation period, but before the L-NAME induced hypertension treatment was started, basal recordings of arterial blood pressure (D0) were measured with a Letica 5002 equipment.

Animals were treated according experimental design as showed in the following table 2. Animals from group 6 were treated with a combined oral gavage of pomegranate an olive fruit extracts, in order to evaluate a possible synergic effect of both extracts in the treatment of the hypertension.

**Table 2.**

| GROUP | Intraperitoneal injection | Oral gavages | Remarks |
|---|---|---|---|
| 1 | sterile saline solution | No | Injection volume similar to groups 2 to 7 |
| 2 | 40 mg L-NAME/Kg | No | |
| 3 | 40 mg L-NAME/Kg | 80 mg punicalagins/ Kg | punicalagins into water |
| 4 | 40 mg L-NAME/Kg | 160 mg PUN/ Kg | punicalagins into water |
| 5 | 40 mg L-NAME/Kg | 5mg hydroxytyrosol/ Kg | Hydroxityrosol into fortified extra virgin olive oil |
| 6 | 40 mg L-NAME/Kg | 80 mg PUN/ Kg + 5mg hydroxytyrosol/ Kg | punicalagins (into water) together with 5 mg of HT (into fortified extra virgin olive oil) per kg. |
| 7 | 40 mg L-NAME/Kg | 100 mg Captopril/ Kg | Positive control: 100 mg of captopril (dissolved in water) |

### 7.4. Cronogramme of activities

In order to achieve rats to be in the habit, measurements of arterial blood pressure using the recording of the pulsations of the tail artery with a Letica 5002 equipment were made during the acclimatation period. Cronogrammes of experimental measurements/treatments were summarized in the following table:

**Table 3.**

| Day | Measurements of arterial blood pressure | Intraperitoneal injection of L-NAME or sterile saline solution | Oral gavages |
|---|---|---|---|
| 0 | Yes | Yes | No |
| 1 | No | Yes | Yes |
| 2 | Yes | Yes | Yes |
| 3 | Yes | Yes | Yes |
| 4 | Yes | Yes | Yes |
| 5 | No | Yes | Yes |
| 6 | No | Yes | Yes |
| 7 | Yes | Yes | Yes |
| 8 | No | Yes | Yes |
| 9 | No | Yes | Yes |
| 10 | Yes | Yes | Yes |

### 7.4. Determination of arterial blood pressure.

Arterial blood pressure was measured with a periodicity according the above cronogramme, by the tail-cuff method. Before the measurement, the rats were kept at 37 °C for 10 min to make the pulsations of the tail artery detectable. The equipment used in the present study, LE 5002 (Letica, Hospitalet, Barcelona, Spain), has a high sensitivity pulse transducer coupled with an accurate microprocessor program, and allow us accurate measurements of arterial blood pressure. The arterial blood pressure measurements were performed at the same time of the day in order to avoid any influence of the circadian cycle.

### 7.5. Statistical analysis

The data are expressed as means±standard error of the means (S.E.M.), and were analyzed by one-way ANOVA. Differences between the groups were assessed by the Tukey test. Differences were considered significant when P-values were <0.05. The data was analysed using Sigma Stat software (Version 2.03).

### 7.6. Discussion.

Arterial hypertension is among risk factors associated with cardiovascular diseases. We have studied the effect that pomegranate extract has on arterial blood pressure, and compared it with control groups without treatment with pomegranate extract. We conclude pomegranate extract, and the regular administration of the nutritional product containing a pomegranate extract showed antihypertensive activity which, to a greater or lesser extent, prevents the occurrence of cardiovascular events and therefore may well be considered as a health promoting dietary supplement and may be a successful strategy to produce functional foods and beverages with antihypertensive activity.

## Claims

1. A nutritional product which is a functional food or functional beverage comprising pomegranate extract to provide at least 5 mg of punicalagins per serving of said product.

2. A nutritional product according to claim 1, wherein said pomegranate extract has a punicalagins content of not less than 5 % w/w, being purity of such punicalagins not less than 55 %, and ellagic acid content of not more than 3 % (w/w), a total phenols content of not less than 20 % w/w, (expressed as gallic acid equivalent), a water solubility of not less than 3 % w/v (30 g per litre), a residual solvent content of 0 ppm, and a total sugar content of not more than 500 ppm.

3. A nutritional product according to claim 1, wherein said pomegranate extract has a punicalagins content of not less than 30 % w/w, being purity of such punicalagins not less than 70 %, and ellagic acid content of not more than 2 % (w/w), a total phenols content of not less than 30 % w/w, (expressed as gallic acid equivalent), a water solubility of not less than 7 % w/v (70 g per litre), a residual solvent content of 0 ppm, and a total sugar content of not more than 300 ppm.

4. A nutritional product according to claim 1, wherein said pomegranate extract has a punicalagins content of not less than 50 % w/w, the purity of such punicalagins being not less than 80 %, and ellagic acid content of not more than 1.5 % (w/w), a total phenols content of not less than 35 % w/w, (expressed as gallic acid equivalent), a water solubility of not less than 10 % w/v (100 g per litre), a residual solvent content of 0 ppm, and a total sugar content of not more than 50 ppm.

5. A nutritional product according to any previous claim, wherein said functional beverage is selected from a non-alcoholic drink, a fermented alcoholic drink or a hot beverage selected from, fruit-flavoured beverage, orange flavoured beverage, lemon-lime flavoured beverage, root beer, cola, fruit juice, fruit-flavoured, or fruit-containing beverage, vegetable juice or vegetable containing beverage, sport drink, energy drink, flavoured water, beverages comprising a dairy component, horchata, beer, beer-type sparkling drink wine, wine-type drink, coffee, coffee-based beverages, tea, tea-based beverages, infusions, to name a few, technologically modified derivatives of the above mentioned beverages or mixtures of two or more of the same.

6. A nutritional product according to any claim 1 to 4, wherein said functional food is selected from a dairy product such us milk, milk shake, fermented milk, flavoured milk, yogurts, ice creams, cheeses, to name a few, technologically modified derivatives of the above mentioned dairy product or mixtures of two or more of the same.

7. A nutritional product according to any claim 1 to 4, wherein said functional food is selected from a vegetable, marine or fish oils obtained from various sources such us olive (extra virgin olive oil, virgin olive oil, lampante olive oil, refined olive oil, crude olive-pomace oil, refined olive-pomace oil), sunflower, corn, soya, flax seed, almond, canola, safflower, palm, coconut, rapeseed, algae, krill, menhaden, anchovy, tuna to name a few, technologically modified derivatives of the above mentioned dairy product or mixtures of two or more of the same.

8. A nutritional product according to any claim 1 to 4, wherein said functional food is selected from a meat or poultry product such us, chicken, turkey, duck, pork, beef, prepared as sausages, smoke-dried or cold meat, such as cured ham, boiled ham, smoked ham, mortadella, salami, pâté, canned precooked meats, technologically modified derivatives of the above mentioned meat or poultry products or mixtures of two or more of the same.

9. A nutritional product according to any claim 1 to 4, wherein said functional food is selected from a bakery product or a pasta-based product such us breads, bagels, biscuits, cookies, cakes, pastries, pies, macaroni, spaghetti, technologically modified derivatives of the above mentioned bakery or pasta-based products or mixtures of two or more of the same.

10. A nutritional product according to any claim 1 to 4, wherein said functional food is selected from a vegetable or fruit conserve such as canned tomatoes, canned artichokes, canned pineapple, canned peach, jams, marmalades, jellies, pickles, technologically modified derivatives of the above mentioned vegetable or fruit conserves or mixtures of two or more of the same.

11. A nutritional product according to any claim 1 to 4, wherein said functional food is selected from canned seafood or canned fish such as shrimp, lobster, squid, crab, mussel, cockle, tuna, sardine, technologically modified derivatives of the above mentioned canned seafood or fish or mixtures of two or more of the same.

12. A nutritional product according to any claim 1 to 4, wherein said functional food is selected from a snack or sweet such as almonds, peanuts, pistachios, walnuts, popcorns, chocolates, chewing gums, candies to name a few, technologically modified derivatives of the above mentioned snacks or sweets or mixtures of two or more of the same.

13. A nutritional product according to any claim 1 to 4, which is a pet functional food.

14. A pomegranate extract having a punicalagins content of not less than 5 % w/w, being purity of such punicalagins not less than 55 %, and ellagic acid content of not more than 3 % (w/w), a total phenols content of not less than 20 % w/w, (expressed as gallic acid equivalent), a water solubility of not less than 3 % w/v (30 g per litre), a residual solvent content of 0 ppm, and a total sugar content of not more than 500 ppm.

15. A pomegranate extract having a punicalagins content of not less than 30 % w/w, being purity of such punicalagins not less than 70 %, and ellagic acid content of not more than 2 % (w/w), a total phenols content of not less than 30 % w/w, (expressed as gallic acid equivalent), a water solubility of not less than 7 % w/v (70 g per litre), a residual solvent content of 0 ppm, and a total sugar content of not more than 300 ppm.

16. A pomegranate extract having a punicalagins content of not less than 50 % w/w, being purity of such punicalagins not less than 80 %, and ellagic acid content of not more than 1.5 % (w/w), a total phenols content of not less than 35 % w/w, (expressed as gallic acid equivalent), a water solubility of not less than 10 % w/v (100 g per litre), a residual solvent content of 0 ppm, and a total sugar content of not more than 50 ppm.

17. A process for preparing nutritional products including functional foods, functional beverages and functional pet foods according any claim 1 to 13 containing at least 5 mg of punicalagins per serving, which comprises adding pomegranate extract according to any claim from 14 to 16, in the production process.

18. A composition comprising an extract of any one of claims 14 to 16, wherein the composition is in a form selected from tablets, granules, suspensions, solutions, emulsions, capsules, powders, suppository, chewing gum, candy and the like.

19. The use of a pomegranate extract according to any claim 14 to 16 as a dietary supplement.

20. The use of extract according to any claim 14 to 16 for the manufacture of a medicament and/or a composition for the prevention or treatment of hypercholesterolemia.

21. The use of an extract according to any claim 14 to 16 for the manufacture of a medicament and/or a composition for the prevention or treatment of arterial hypertension.

22. A functionalized nutritional product according any claim 1 to 13 comprising from 0.1 % in weight to 5% in weight of the added extract of claim 14.

23. A functionalized nutritional product according any claim 1 to 13 comprising from 0.01% in weight to 1.0% in weight of the added extract of claim 15.

24. A functionalized nutritional product according any claim 1 to 13 comprising from 0.01% in weight to 0.5% in weight of the added extract of claim 16.

25. A functionalized nutritional product according any claim 1 to 13 and 22 to 24, further comprising hydroxytyrosol.

26. Use of a functionalized nutritional product according to any claim 22 to 25 for the manufacture of a composition for the prevention or treatment of hypercholesterolemia.

27. A functionalized nutritional product according to any claim 22 to 25 for the manufacture of a composition for use in the prevention or treatment of arterial hypertension.

28. Extract according to any claim 14 to 16 for the prevention or treatment of hypercholesterolemia.

29. Extract according to any claim 14 to 16 for the prevention or treatment of arterial hypertension.

30. Nutritional product according to any claim 22 to 25 for the prevention or treatment of hypercholesterolemia.

31. Nutritional product according to any claim 22 to 25 for the prevention or treatment of arterial hypertension.
